(19) Europäisches Patentamt
European Patent Office
Office européen des brevets

(11) EP 3 332 764 A1

# (12) EUROPEAN PATENT APPLICATION
published in accordance with Art. 153(4) EPC

(43) Date of publication:
**13.06.2018 Bulletin 2018/24**

(21) Application number: **16833106.4**

(22) Date of filing: **04.08.2016**

(51) Int Cl.:
***A61K 8/81*** *(2006.01)* ***A61Q 11/00*** *(2006.01)*

(86) International application number:
**PCT/JP2016/072955**

(87) International publication number:
**WO 2017/022827 (09.02.2017 Gazette 2017/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.08.2015 JP 2015155410**

(71) Applicant: **NOF Corporation**
**Shibuya-ku**
**Tokyo 150-6019 (JP)**

(72) Inventors:
- **MIYAMOTO,Koji**
**Kawasaki-shi**
**Kanagawa 210-0865 (JP)**
- **YAMAMOTO,Nobuyuki**
**Kawasaki-shi**
**Kanagawa 210-0865 (JP)**
- **SAKURAI,Shunsuke**
**Kawasaki-shi**
**Kanagawa 210-0865 (JP)**
- **SHIMAMURA,Yoshihisa**
**Kawasaki-shi**
**Kanagawa 210-0865 (JP)**

(74) Representative: **Wills, Andrew Jonathan**
**Mewburn Ellis LLP**
**City Tower**
**40 Basinghall Street**
**London EC2V 5DE (GB)**

## (54) AGENT FOR PREVENTING FORMATION OF BIOFILM, AND COMPOSITION FOR ORAL USE

(57) Provided is a biofilm formation-preventing agent that improves the sustainability of an effect of a drug, and keeps an oral environment satisfactory in a sustainable manner, without an increase in drug concentration or treatment time to prevent the maturation of dental plaque into a biofilm, and a composition for oral use containing the biofilm formation-preventing agent. More specifically, the biofilm formation-preventing agent includes a copolymer having a weight-average molecular weight of from 10,000 to 5,000,000 and containing: 10 mol% to 90 mol% of a constituent unit (A) based on 2-(meth)acryloyloxyethyl phosphorylcholine; and 90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth) acrylic monomer, and a constituent unit (B3) based on a (meth)acrylamide-based monomer.

EP 3 332 764 A1

## Description

### Technical Field

The present invention relates to a biofilm formation-preventing agent and a composition for oral use containing the biofilm formation-preventing agent.
The present application claims priority from Japanese Patent Application No. 2015-155410, which is incorporated herein by reference.

### Background Art

An oral environment generally refers to the state of the entire oral cavity including teeth, gums, a tongue, an oral mucosa, and the like, and the deterioration of the oral environment is responsible for, for example, the occurrence of a mouth odor, a decayed tooth, or a periodontal disease, thereby leading to a reduction in quality of life. The deterioration of the oral environment starts from the formation of dental plaque serving as an accumulated product in which bacteria become a solid mass, and the dental plaque is matured over a certain time period to lead to the formation of a biofilm serving as the life assembly of a plurality of bacteria. Although the dental plaque can be relatively easily removed by tooth brushing, the biofilm is hard to remove by the tooth brushing. Pathogenic bacteria for caries and a periodontal disease produced from the biofilm may cling to a heart valve or the like to proliferate, thereby causing bacterial endocarditis, and in an aged person, the pathogenic bacteria may enter his or her bloodstream to cause a circulatory disturbance, such as a coronary disease, a cardiac disease, or the attack of cardiac infarction.
Because of the foregoing, a composition for oral use obtained by blending a bactericidal component, an anti-inflammatory component, and a blood circulation-promoting component is used for keeping the oral environment satisfactory in a sustainable manner to prevent the maturation of the dental plaque into the biofilm. However, the time period for which a biofilm formation-preventing agent or a drug to be incorporated into the composition for oral use is in contact with an affected part is short because after the composition has been used in an oral cavity, the composition is spewed or the inside of the oral cavity is rinsed with tap water in ordinary cases. Further, even when the drug is in contact with the affected part, the drug may be flowed by saliva. Accordingly, there is a problem in that even when a drug in accordance with a purpose is used, an effect thereof is not sufficiently exhibited.
In view of the foregoing, in order that an effect of a drug may be sufficiently exhibited, measures have been taken to increase a drug concentration or to lengthen a treatment time. However, some drugs cause inflammation as a side effect when their drug concentrations are increased, and the lengthening of the treatment time is inconvenient for a user. Accordingly, the above-mentioned measures are not necessarily sufficient.
A technology intended to cause a drug to remain in an affected part through the impartment of viscosity to a composition for oral use instead of a change in drug concentration or treatment time has been opened to the public. In, for example, Patent Literature 1, there is a disclosure of a method involving improving the retentivity of a disinfectant or a vitamin in an oral cavity with a nonionic water-soluble polymer having viscosity. The method involves a problem in that the impartment of the viscosity to the composition for oral use gives a user a heavy feeling of use, and a problem in that the dosage form of the composition is limited because certain viscosity is needed.
In addition, as a technology for improvements in adsorptivity and sustained releasability, in Patent Literature 2, there is a disclosure of a technology concerning a composition for oral use obtained by combining a water-soluble monofluoro phosphorylated polymer compound excellent in retentivity of a fluoride and in releasability of the fluoride, and a surfactant. However, the technology involves a problem in that its applications are limited to improvements in retentivity and releasability of the fluoride serving as a specific drug.
Meanwhile, a composition for oral use using a phosphorylcholine group-containing polymer (PC polymer) has been known. In Patent Literature 3, there is a disclosure of a technology concerning a composition for oral use that keeps the inside of an oral cavity wet, and in Patent Literature 4, there is a disclosure of a technology concerning a microbial adhesion-preventing agent for oral use characterized by containing the PC polymer as a microbial adhesion-preventing component, a water-soluble polysaccharide as a binder component, and a poly(meth)acrylic acid derivative as a solubilizing component.
However, in Patent Literature 3, there is no description or suggestion concerning the use of the PC polymer as a biofilm formation-preventing agent. In addition, the technology of Patent Literature 4 is a technology by which the adhesion of a microbe in an oral cavity can be prevented, but in the literature, there is no description concerning a biofilm formation-preventing agent that suppresses coaggregation in which any other microbe adheres to a microbe that has already adhered to the oral cavity.
Thus, a composition for oral use that keeps an oral environment satisfactory in a sustainable manner without an increase in drug concentration or treatment time to prevent the maturation of dental plaque into a biofilm has been required.

Citation List

Patent Literature

[PTL 1] JP 2008-120753 A
[PTL 2] JP 2009-102283 A
[PTL 3] JP 2006-273767 A
[PTL 4] JP 2011-153101 A

Summary of Invention

Technical Problem

As described above, an object of the present invention is to provide a biofilm formation-preventing agent that improves the sustainability of an effect of a drug, and keeps an oral environment satisfactory in a sustainable manner, without an increase in drug concentration or treatment time to prevent the maturation of dental plaque into a biofilm, and a composition for oral use containing the biofilm formation-preventing agent.

Solution to Problem

The inventors of the present invention have made extensive investigations for solving the above-mentioned problems, and as a result, have found that when a (meth)acrylic copolymer containing a phosphorylcholine group having a specific structure is used as a biofilm formation-preventing agent with a view to suppressing coaggregation in which any other microbe adheres to a microbe that has already adhered to an oral cavity, the amount of a drug to be adsorbed to a tooth surface can be controlled and the sustained release of the drug can be achieved. Thus, the inventors have completed the present invention.

That is, the present invention is as described below.

1. Abiofilm formation-preventing agent, including a copolymer having a weight-average molecular weight of from 10,000 to 5,000, 000 and containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

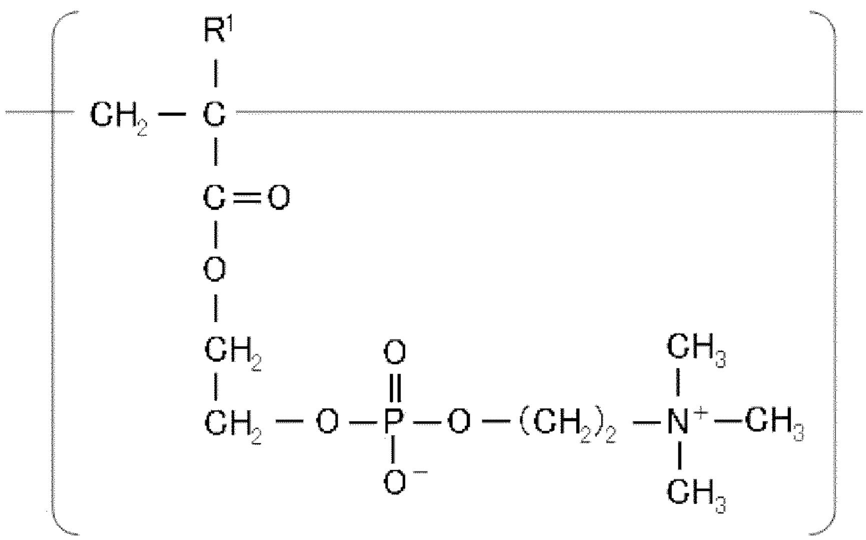

··· (A)

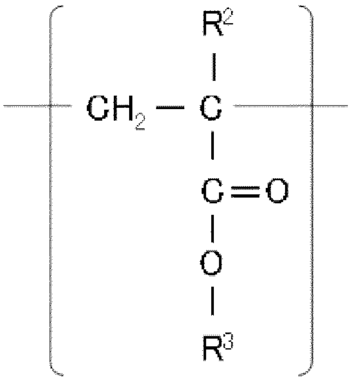

···(B1)

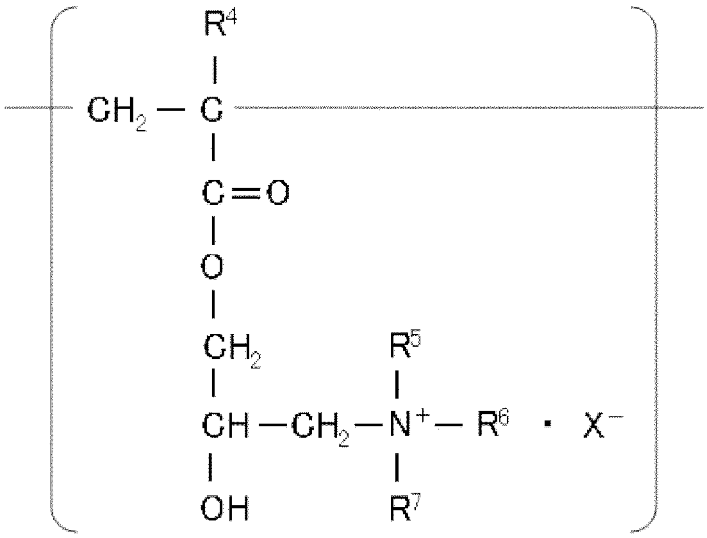

···(B2)

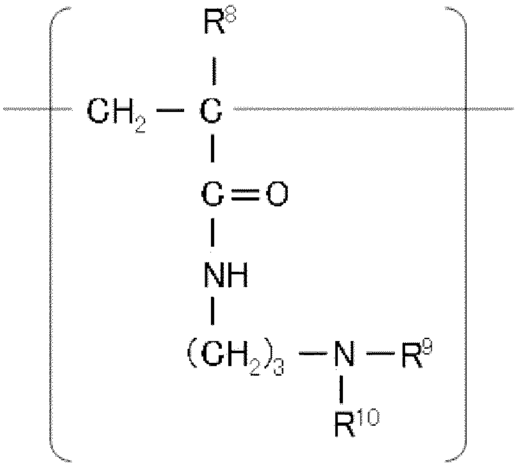

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

2. A biofilm formation-preventing agent according to item 1, in which the constituent unit (A) includes a constituent unit based on 2- (meth) acryloyloxyethyl phosphorylcholine, and the constituent unit (B1) includes a constituent unit

based on butyl methacrylate.

3. A biofilm formation-preventing agent according to item 1, in which the constituent unit (A) includes a constituent unit based on 2- (meth) acryloyloxyethyl phosphorylcholine, and the constituent unit (B1) includes a constituent unit based on stearyl methacrylate.

4. A biofilm formation-preventing agent according to item 1, in which the constituent unit (A) includes a constituent unit based on 2- (meth) acryloyloxyethyl phosphorylcholine, and the constituent unit (B2) includes a constituent unit based on 2-hydroxy-3-methacryloyloxypropyltrimethylammonium chloride.

5. A biofilm formation-preventing agent according to item 1, in which the constituent unit (A) includes a constituent unit based on 2-(meth)acryloyloxyethyl phosphorylcholine, the constituent unit (B1) includes a constituent unit based on stearyl methacrylate, and the constituent unit (B3) includes a constituent unit based on N,N-dimethylaminopropyl acrylamide.

6. A composition for oral use, including: the biofilm formation-preventing agent of any one of Items 1 to 5; and one or two or more kinds of drugs selected from the group consisting of a disinfectant (C), an anti-inflammatory agent (D), and a vitamin (E) .

7. A biofilm formation-preventing method, including the following step:

administering, to an oral cavity of a mammal including a human, a biofilm formation-preventing agent or a composition for oral use including the biofilm formation-preventing agent, the biofilm formation-preventing agent including a copolymer having a weight-average molecular weight of from 10,000 to 5,000,000 and containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

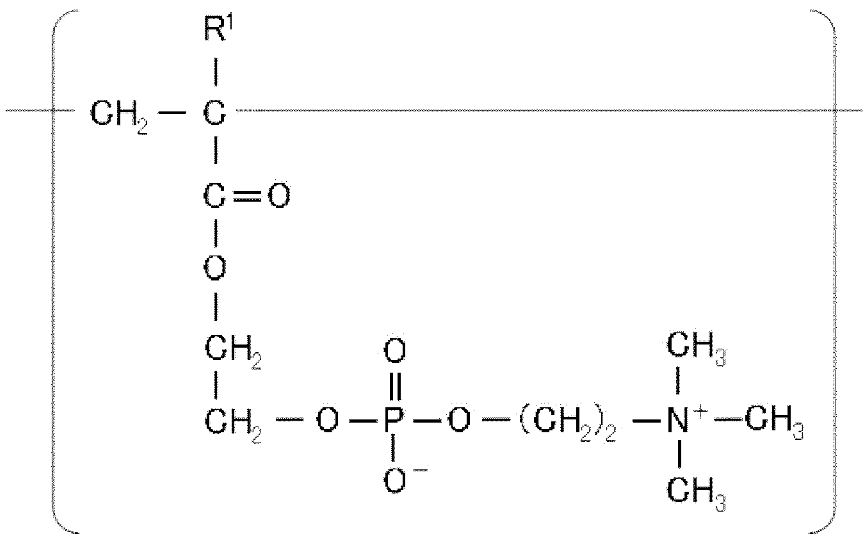

· · · (A)

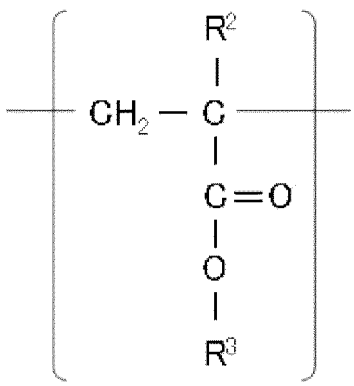

· · · (B1)

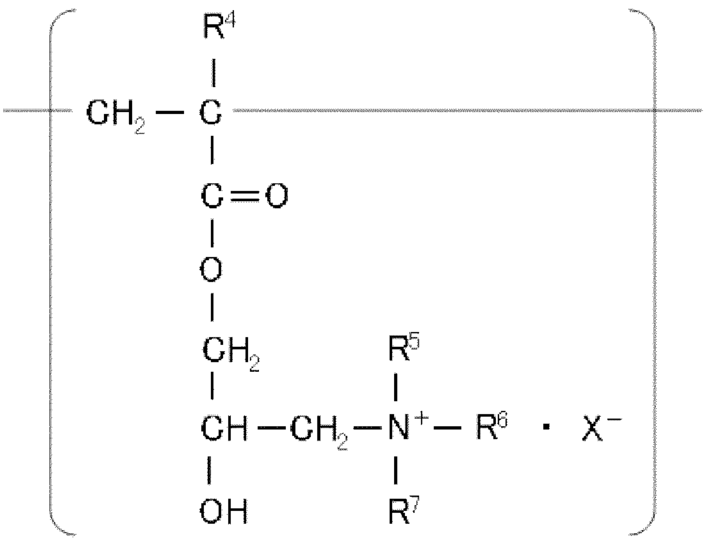

···(B2)

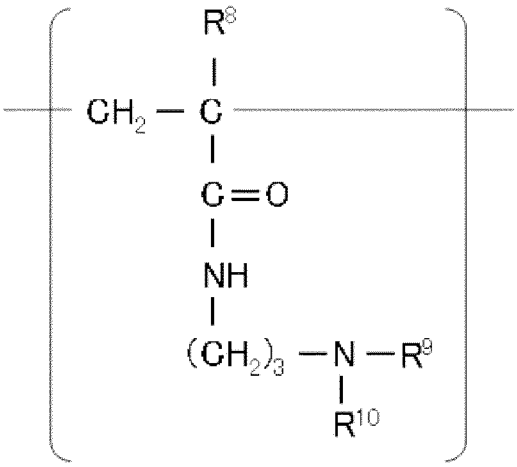

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

8. A copolymer for preventing biofilm formation, the copolymer having a weight-average molecular weight of from 10, 000 to 5, 000, 000 and containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

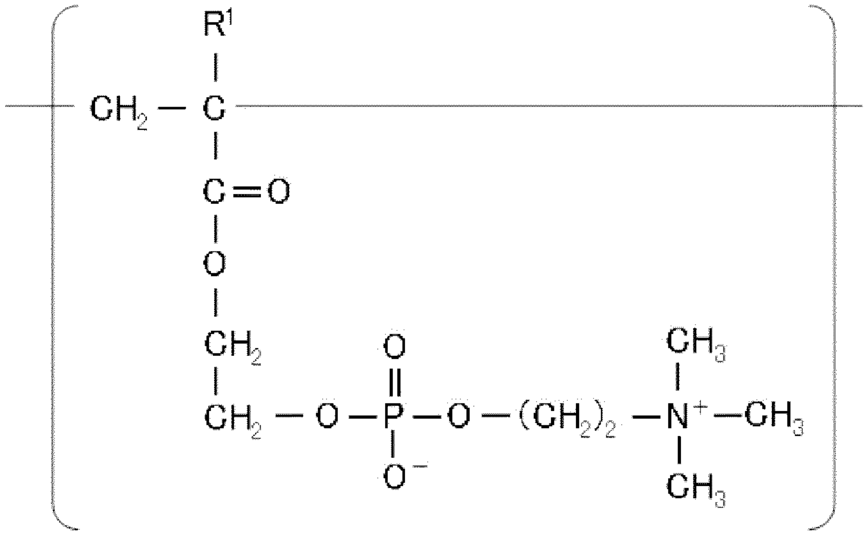

・・・(A)

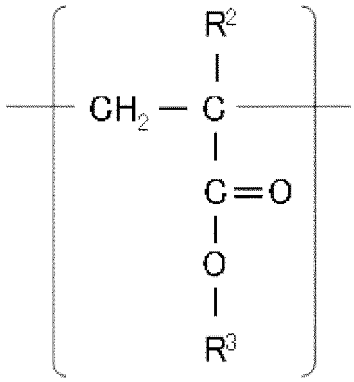

・・・(B1)

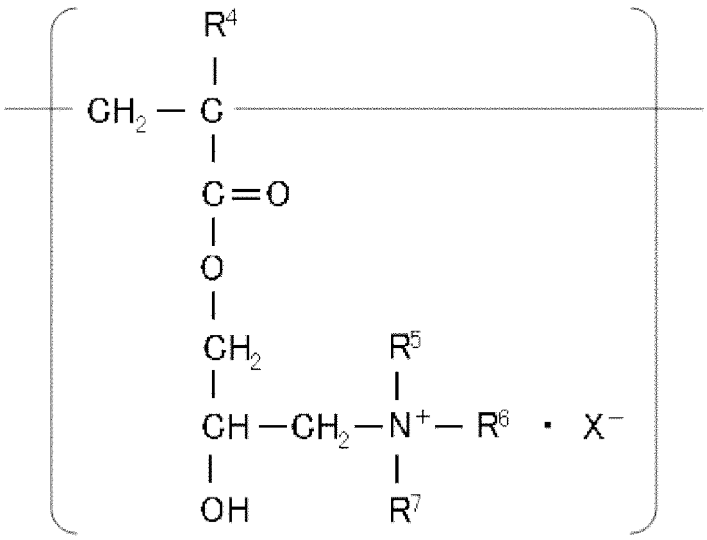

・・・(B2)

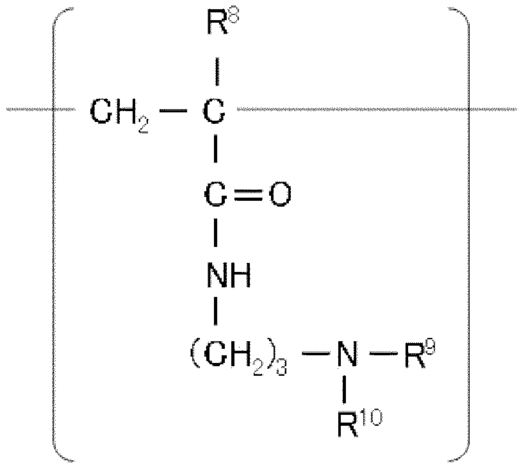

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

9. A use of a copolymer for producing a biofilm formation-preventing agent, the copolymer having a weight-average molecular weight of from 10,000 to 5,000,000 and containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

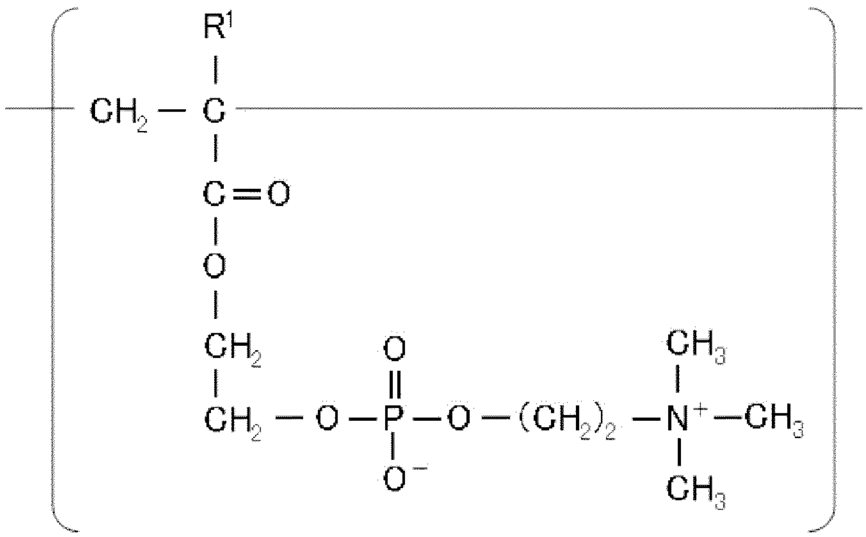

···(A)

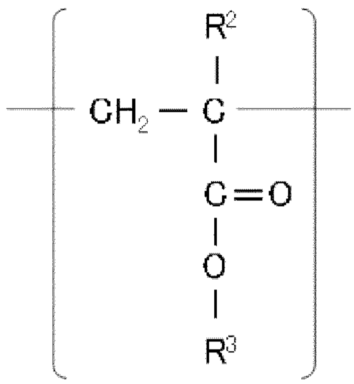

···(B1)

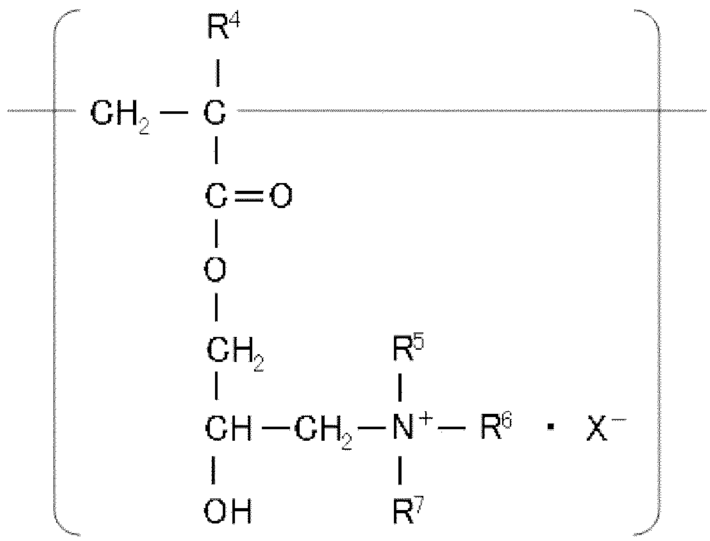

···(B2)

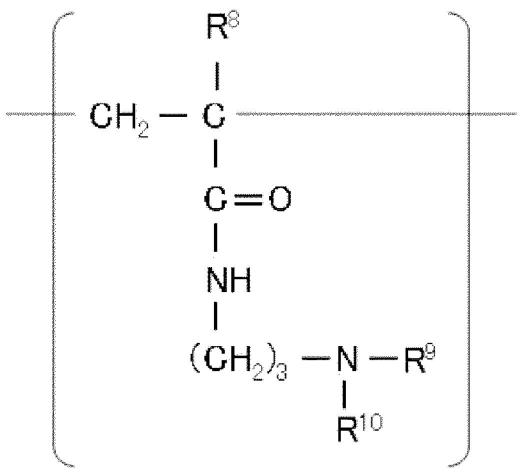

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

Advantageous Effects of Invention

The biofilm formation-preventing agent of the present invention can prevent the maturation of dental plaque into a biofilm through the biofilm formation-preventing effect of its (meth) acrylic copolymer containing a phosphorylcholine group. Further, the composition for oral use of the present invention contains the biofilm formation-preventing agent and a drug. Accordingly, the amount of the drug to be adsorbed to a tooth surface can be controlled, and hence a side effect exhibited by the drug can be reduced. In addition, an effect of the drug can be sustained for a long time period by the sustained release of the adsorbed drug. Further, the suppression of coaggregation in which any other microbe adheres to a microbe that has already adhered to an oral cavity, and the prevention of stickiness in the oral cavity can be achieved by further improving the biofilm formation-preventing effect.

Description of Embodiments

The present invention is described in more detail below.

< (1) Biofilm Formation-preventing Agent of the Present Invention>

A biofilm formation-preventing agent of the present invention is formed of a copolymer containing a constituent unit (A) based on 2-(meth)acryloyloxyethyl phosphorylcholine, and at least one kind of constituent unit selected from the group consisting of a constituent unit (B1) based on an alkyl group-containing (meth) acrylic monomer, a constituent unit (B2) based on a quaternary ammonium group-containing (meth) acrylic monomer, and a constituent unit (B3) based on a (meth)acrylamide-based monomer. More specifically, the biofilm formation-preventing agent is formed of a copolymer containing the constituent unit (A) based on 2-(meth)acryloyloxyethyl phosphorylcholine, and the constituent unit (B1) based on the alkyl group-containing (meth) acrylic monomer, the constituent unit (B2) based on the quaternary ammonium group-containing (meth)acrylic monomer, or the constituent unit (B3) based on the (meth)acrylamide-based monomer.

<Constituent Unit (A) based on 2-(Meth)acryloyloxyethyl Phosphorylcholine>

The constituent unit (A) based on 2-(meth)acryloyloxyethyl phosphorylcholine is more specifically represented by the following formula (A), and is obtained by the polymerization of a monomer represented by the formula (A').

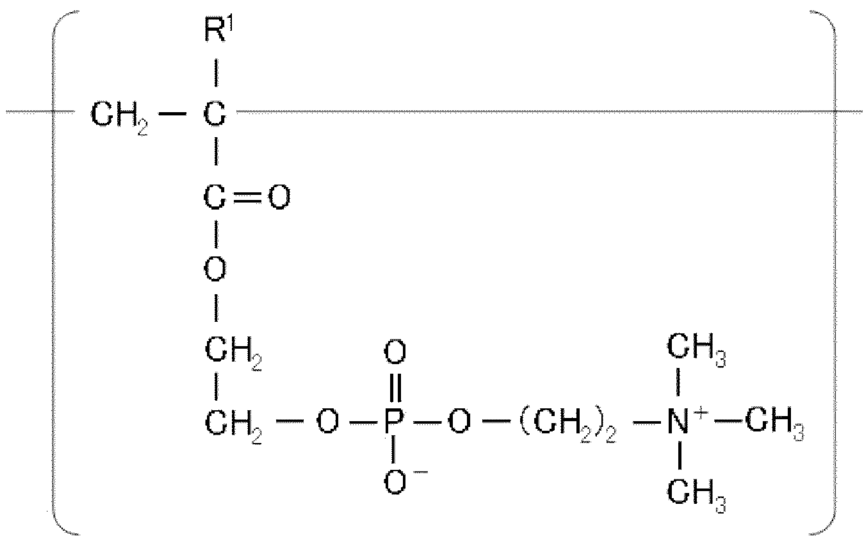

···(A)

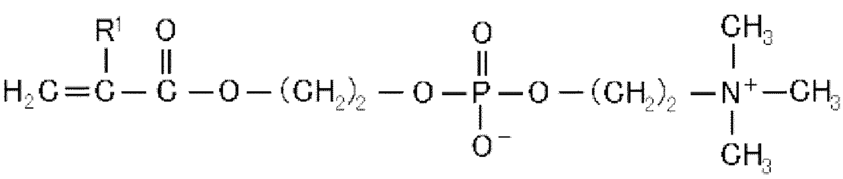

···(A')

In each of the formula (A) and the formula (A'), $R^1$, which may represent any one of a hydrogen atom and a methyl group, preferably represents a methyl group.

When the copolymer to be used in the present invention has the constituent unit (A) in its molecular chain, excess adsorption of a drug can be controlled. The content of the constituent unit (A) in the copolymer to be used in the present invention is from 10 mol% to 90 mol%, preferably from 20 mol% to 90 mol%, more preferably from 30 mol% to 90 mol%. When the content is excessively low, the excess adsorption cannot be suppressed (an excess adsorption-suppressing effect reduces), and when the content is excessively high, the constituent unit itself flows out into water owing to its super hydrophilicity, and hence the effect reduces.

A suitable example of the 2-(meth)acryloyloxyethyl phosphorylcholine is 2-methacryloyloxyethyl phosphorylcholine.

In the present invention, the term " (meth) acryl" means acryl or methacryl, the term "(meth)acryloyl" means acryloyl or methacryloyl, and the term "(meth)acrylate" means acrylate or methacrylate.

<Constituent Unit (B1) based on Alkyl Group-containing (Meth)acrylic Monomer>

The constituent unit (B1) based on the alkyl group-containing (meth)acrylic monomer is more specifically represented by the following formula (B1), and is obtained by the polymerization of a monomer represented by the formula (B1').

When the copolymer to be used in the present invention has the constituent unit (B1) in its molecular chain, the property by which the copolymer is adsorbed to a tooth surface can be further improved.

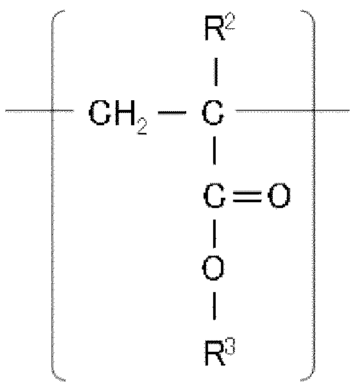

···(B1)

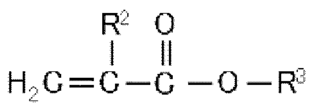

···(B1')

In each of the formula (B1) and the formula (B1'), $R^2$, which may represent any one of a hydrogen atom and a methyl group, preferably represents a methyl group, and $R^3$ may represent any one of linear and branched alkyl groups each having 4 to 18 carbon atoms .

Examples of the linear alkyl group having 4 to 18 carbon atoms include a n-butyl group, a n-pentyl group, a n-hexyl group, a n-heptyl group, a n-octyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, and a n-octadecyl group. Examples of the branched alkyl group having 4 to 18 carbon atoms include a t-butyl group, an isobutyl group, an isopentyl group, a t-pentyl group, a neopentyl group, an isohexyl group, an isoheptyl group, an isooctyl group, an isononyl group, an isodecyl group, an isoundecyl group, an isododecyl group, an isotridecyl group, an isotetradecyl group, an isopentadecyl group, an isohexadecyl group, an isoheptadecyl group, and an isooctadecyl group.

$R^3$ more preferably represents a n-butyl group, a n-dodecyl group, a n-octadecyl group, or the like.

Suitable examples of the alkyl group-containing (meth) acrylic monomer include butyl (meth)acrylate, lauryl (meth)acrylate, stearyl (meth)acrylate, and 2-ethylhexyl (meth)acrylate.

<Constituent Unit (B2) based on Quaternary Ammonium Group-containing (Meth)acrylic Monomer>

The constituent unit (B2) based on the quaternary ammonium group-containing (meth)acrylic monomer is more specifically represented by the following formula (B2), and is obtained by the polymerization of a monomer represented

by the formula (B2').

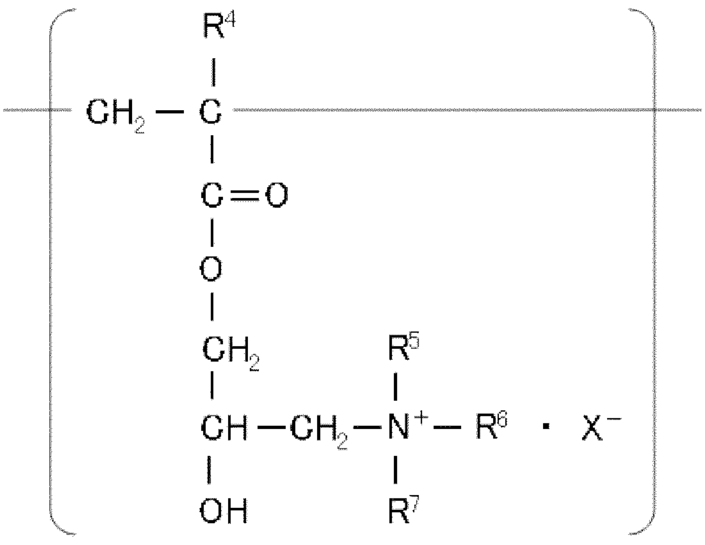

・・・(B2)

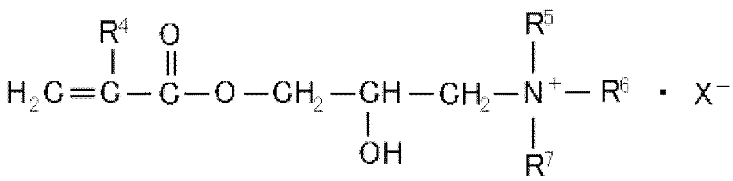

・・・(B2')

In each of the formula (B2) and the formula (B2'), $R^4$, which may represent any one of a hydrogen atom and a methyl group, preferably represents a methyl group, and $R^5$, $R^6$, and $R^7$ each independently represent a hydrogen atom or an alkyl group having 1 to 8 (more preferably 1 to 3) carbon atoms, and the alkyl group may be any one of linear, branched, and cyclic alkyl groups.

Specifically, $R^5$, $R^6$, and $R^7$ each represent a methyl group, an ethyl group, a propyl group, an isopropyl group, a n-butyl group, an isobutyl group, a sec-butyl group, a tert-butyl group, a n-pentyl group, an isopentyl group, a n-hexyl group, an isohexyl group, a n-heptyl group, an isoheptyl group, a n-octyl group, an isooctyl group, a cyclopropyl group, a cyclobutyl group, a cyclopentyl group, and a cyclohexyl group.

$R^5$, $R^6$, and $R^7$ each represent preferably a methyl group, an ethyl group, a propyl group, or an isopropyl group, more preferably a methyl group.

$X^-$ represents, for example: a halogen ion, such as a fluorine ion, a bromine ion, or a chlorine ion (a chloride ion) ; or an acid residue, such as a sulfate ion or a methylsulfate ion. Of those, a halogen ion is preferred.

A suitable example of the quaternary ammonium group-containing (meth)acrylic monomer is 2-hydroxy-3-methacryloyloxypropyltrimethylammonium chloride.

When the copolymer to be used in the present invention has the constituent unit (B2) in its molecular chain, the property by which the copolymer is adsorbed to a tooth surface can be further improved.

<Constituent Unit (B3) based on (Meth) acrylamide-based Monomer>

The constituent unit (B3) based on the (meth) acrylamide-based monomer is more specifically represented by the following formula (B3), and is obtained by the polymerization of a monomer represented by the formula (B3') . When the copolymer to be used in the present invention has the constituent unit (B3) in its molecular chain, the molecular weight of the copolymer is increased, and hence the adhesiveness of the copolymer to a tooth surface can be further improved.

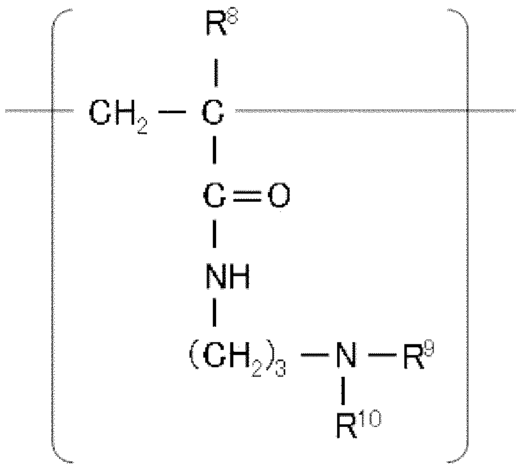

・・・(B3)

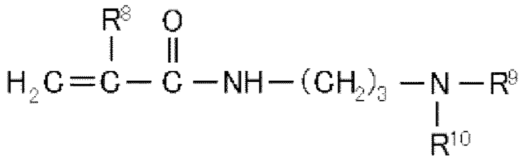

・・・(B3')

In each of the formula (B3) and the formula (B3') , $R^8$, which may represent any one of a hydrogen atom and a methyl group, preferably represents a hydrogen atom, and $R^9$ and $R^{10}$ each independently represent a hydrogen atom or an alkyl group having 1 to 6 carbon atoms, and the alkyl group may be any one of linear, branched, and cyclic alkyl groups. Specific examples thereof include a methyl group, an ethyl group, a propyl group, an isopropyl group, a n-butyl group, an isobutyl group, a sec-butyl group, a tert-butyl group, a n-pentyl group, an isopentyl group, a n-hexyl group, an isohexyl group, a cyclopropyl group, a cyclobutyl group, a cyclopentyl group, and a cyclohexyl group.

$R^9$ and $R^{10}$ each represent preferably a hydrogen atom, a methyl group, an ethyl group, a propyl group, or an isopropyl group, more preferably a methyl group.

A suitable example of the (meth) acrylamide-based monomer may be N,N-dimethylaminopropyl acrylamide.

The copolymer to be used in the present invention has at least one constituent unit selected from the group consisting of the constituent units (B1), (B2), and (B3) in its molecular chain. The copolymer to be used in the present invention may have only one constituent unit selected from the group consisting of the constituent units (B1), (B2), and (B3) in the molecular chain, may have two constituent units selected from the group consisting of the constituent units (B1), (B2), and (B3) {e.g., the constituent units (B1) and (B2), the constituent units (B1) and (B3), or the constituent units (B2) and (B3)} therein, or may have all the constituent units (B1), (B2), and (B3) therein.

When the copolymer to be used in the present invention has the constituent units (B1) to (B3) in its molecular chain, the property by which the phosphorylcholine group-containing polymer remains on a tooth surface can be improved. Further, when the copolymer to be used in the present invention has the constituent unit (A) as well as the constituent units (B1) to (B3) in the same polymer chain, the copolymer has a suppressing effect on excess adsorption of a drug and the sustained releasability of the drug.

The content of the constituent units (B1) to (B3) in the copolymer to be used in the present invention {when the copolymer contains only one of the constituent units (B1) to (B3), the content of the constituent unit, and when the copolymer contains two or three of the constituent units (B1) to (B3), the total content of the constituent units} is from 10 mol% to 90 mol%, preferably from 10 mol% to 80 mol%, more preferably from 10 mol% to 70 mol%. When the content is excessively low, the copolymer has so high hydrophilicity that the copolymer flows at the time of its use in water and hence its sustained release effect reduces, and when the content is excessively high, the amount of the drug to be adsorbed to a tooth surface cannot be controlled (the drug is excessively adsorbed thereto).

Suitable examples of the combination of the constituent unit (A) and the constituent units (B1) to (B3) to be incorporated into the molecular chain of the copolymer to be used in the present invention are as described below. For convenience, the combinations are described by the names of monomers.

2-(Meth)acryloyloxyethyl phosphorylcholine (A) and butyl methacrylate (B1);
2-(meth)acryloyloxyethyl phosphorylcholine (A) and stearyl methacrylate (B1);
2-(meth)acryloyloxyethyl phosphorylcholine (A) and 2-hydroxy-3-methacryloyloxypropyltrimethylammonium chloride (B2); and

2-(meth)acryloyloxyethyl phosphorylcholine (A), N,N-dimethylaminopropyl acrylamide (B3), and stearyl methacrylate (B1) .

Although the copolymer to be used in the present invention may contain a constituent unit except the constituent unit (A) and the constituent units (B1) to (B3), the copolymer is preferably formed only of the constituent unit (A), and one, two, or three constituent units selected from the group consisting of the constituent units (B1), (B2), and (B3).
A polymer (1) obtained by performing polymerization in conformity with the method of JP 11-035605 A, a polymer (2) obtained by performing polymerization in conformity with the method of JP 2004-196868 A, a polymer (3) obtained by performing polymerization in conformity with the method of JP 2004-196868 A, a polymer (4) obtained by performing polymerization in conformity with the method of JP 2004-189678 A, a polymer (5) obtained by performing polymerization in conformity with the method of JP 2013-018749 A, or the like may be used as the copolymer to be used in the present invention.
The weight-average molecular weight of the copolymer to be used in the present invention is from 10,000 to 5,000,000, preferably from 50,000 to 1,000,000. When the weight-average molecular weight is excessively low, an effect commensurate with the blending amount of the copolymer is not obtained. When the weight-average molecular weight is excessively high, a certain effect is obtained but a feeling of use deteriorates; for example, a filmy feeling becomes excessively strong at the time of the use of the copolymer.
In addition, when the above-mentioned biofilm formation-preventing agent is applied to a composition for oral use, a drug that particularly improves an effect thereof is, for example, a disinfectant (C), an anti-inflammatory agent (D), or a vitamin (E). Examples of those drugs include, but not particularly limited to, the following.
The disinfectant (C) is one or two or more kinds of disinfectants selected from the group consisting of isopropyl methylphenol, dequalinium chloride, benzalkonium chloride, benzethonium chloride, alkyldiaminoethylglycine hydrochloride, chlorhexidine hydrochloride, chlorhexidine gluconate, triclosan, and 1,8-cineol.
The anti-inflammatory agent (D) is one or two or more kinds of disinfectants selected from the group consisting of sodium azulene sulfonate, allantoin, glycyrrhizic acid and salts thereof, and β-glycyrrhizic acid.
The vitamin (E) is one or two or more kinds of vitamins selected from the group consisting of ascorbic acid and salts thereof, pyridoxine hydrochloride, dl-α-tocopherol acetate, and dl-α-tocopherol nicotinate.
The composition for oral use of the present invention can be applied to, for example, a mouthwash, a gargle, a dentifrice, a mouth refrigerant, or a chewable, and its form, such as liquid, viscous liquid, or gel, is not particularly limited.
Further, a drug, a buffer, a moisturizer, a surfactant, an antiseptic disinfectant, a sweetener, a thickener, a solvent, a coloring material, an organic acid, inorganic salts, an antioxidant, a stabilizer, an antiseptic agent, a sequestrant, a flavoring, a taste-masking agent, a cooling agent, or a color material, which is generally used for a composition for oral use, may be blended in the composition for oral use of the present invention as required.
A drug except the disinfectant (C), the anti-inflammatory agent (D), and the vitamin (E) may be incorporated in a pharmaceutically blendable amount into the composition for oral use of the present invention.
Examples of the drug include:

a styptic, such as ε-aminocaproic acid or tranexamic acid;
a hyperesthesia reliever, such as potassium nitrate or aluminum lactate;
an expectorant, such as 1,8-cineol;
an adsorbent, such as zeolite;
a calculus deposition inhibitor, such as disodium dihydrogen pyrophosphate, sodium pyrophosphate, or zinc chloride;
a decayed tooth-preventing agent, such as sodium fluoride or sodium monofluorophosphate;
a mouth odor remover, such as sodium copper chlorophyllin;
a cleaner, such as polyoxyethylene lauryl ether (8 to 10 E.O.) or lauroylsarcosine sodium;
an astringent, such as sodium chloride or 1-menthol; and
a dental plaque remover, such as sodiummonohydrogen phosphate, trisodium phosphate, polyethylene glycol, or polyvinylpyrrolidone.

Examples of the buffer include, but not particularly limited to, citric acid, phosphoric acid, malic acid, and gluconic acid and salts thereof, and the buffer is desirably used in an amount of from 0.01 mass% to 3 mass%.
Examples of the moisturizer include, but not particularly limited to, polyhydric alcohols, such as propylene glycol, butylene glycol, pentylene glycol, dipropylene glycol, polyethylene glycol, xylitol, mannitol, and erythritol, and the moisturizer is desirably used in an amount of from 1 mass% to 50 mass%.
Examples of the surfactant include, but not particularly limited to, a polyoxyethylene hydrogenated castor oil, a sorbitan fatty acid ethylene adduct, a polyglycerin fatty acid ester, an acylamino acid salt, a fatty acid aminopropyl betaine, and a fatty acid amide betaine, and a polyoxyethylene hydrogenated castor oil or a sorbitan fatty acid ethylene adduct is particularly preferably used in an amount of from 0.05 mass% to 2 mass%.

Examples of the antiseptic disinfectant include, but not particularly limited to, polyhexanide hydrochloride, benzoic acid and salts thereof, and parabens, and the blending amount thereof is typically from 0.01 mass% to 1 mass%.

Examples of the sweetener include, but not particularly limited to, saccharin, stevioside, sucrose, aspartame, and an extract of licorice root.

Examples of the thickener include, but not particularly limited to: cellulose-based thickeners, such as methyl cellulose, hydroxyethyl cellulose, hydroxypropyl cellulose, hydroxypropylmethyl cellulose, and carboxymethyl cellulose; and polysaccharides, such as hyaluronic acid and salts thereof, chondroitin sulfate and salts thereof, alginic acid and salts thereof, gellan gum, and xanthan gum.

Examples of the solvent include, but not particularly limited to, purified water and ethanol. Of those, ethanol is particularly preferably used in an amount of from 5.0 mass% to 20 mass%.

The composition for oral use containing the biofilm formation-preventing agent of the present invention may be obtained by dissolving the copolymer to be used in the present invention in water, an alcohol, such as ethanol, or a mixed solution thereof so that its concentration may be from 0.01 mass% to 10.0 mass%. When the concentration of the copolymer is less than 0.01 mass%, a suppressing effect on excess adsorption of a drug and a sustained release effect on the drug are not sufficient, and when the concentration is more than 10.0 mass%, it may be difficult to control the amount of the drug to be adsorbed to a tooth surface.

In addition, the concentration of the copolymer to be used in the present invention is more preferably from 0.05 mass% to 5.0 mass%, still more preferably from 0.5 mass% to 5.0 mass%.

The present invention is also directed to a biofilm formation-preventing method including the following step: administering, to an oral cavity of a mammal including a human, a biofilm formation-preventing agent or a composition for oral use including the biofilm formation-preventing agent, the biofilm formation-preventing agent including a copolymer having a weight-average molecular weight of from 10,000 to 5,000,000, the copolymer containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

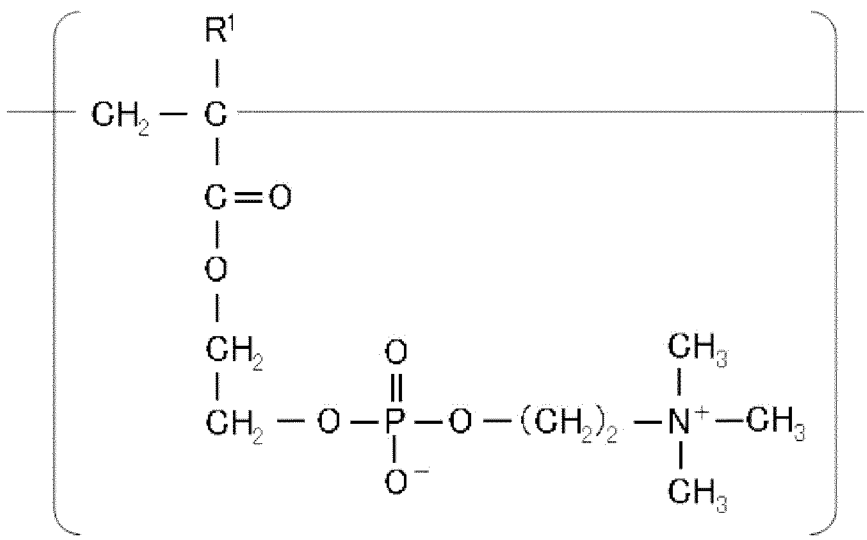

· · · (A)

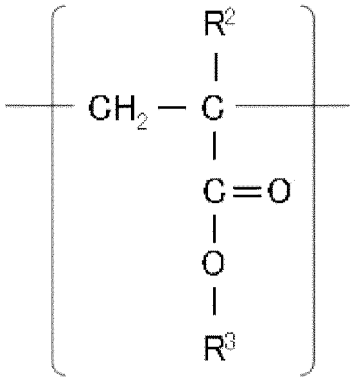

・・・(B1)

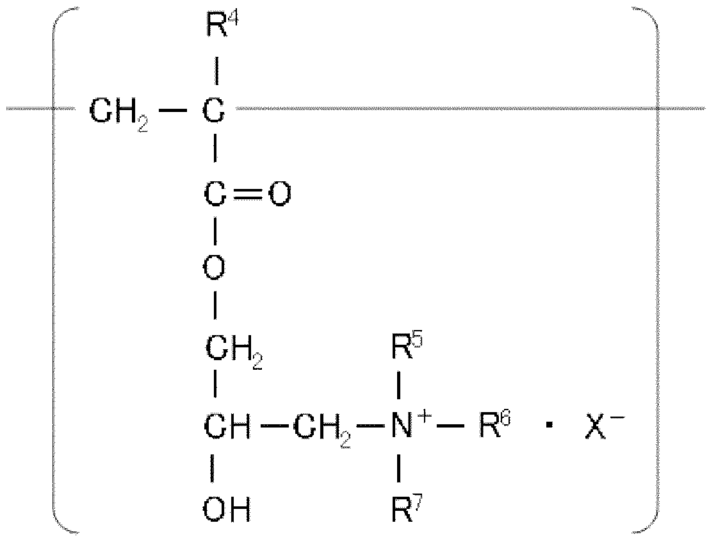

・・・(B2)

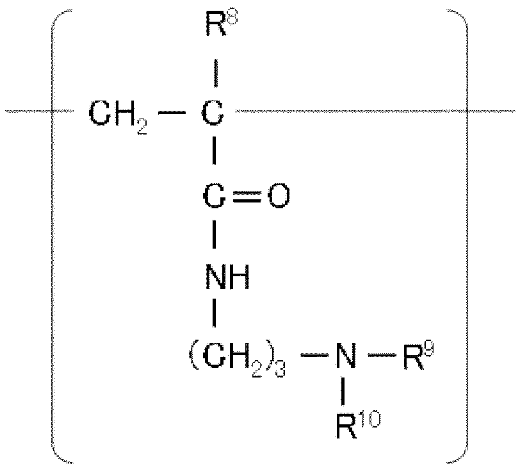

・・・(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

The biofilm formation-preventing method of the present invention, which is not particularly limited, may be, for example, the following mouth washing (gargling) : 15 mL to 25 mL of the composition for oral use containing the biofilm

formation-preventing agent of the present invention is incorporated into a mouth 1 to 10 times, 1 to 8 times, 1 to 6 times, 1 to 4 times, or 1 to 3 times (preferably in the morning, at noon, and in the evening) per day for 30 seconds or more per time.

The biofilm formation-preventing method is directed to, but not particularly limited to, mammals including a human.

The present invention is also directed to a copolymer for preventing biofilm formation, the copolymer having a weight-average molecular weight of from 10,000 to 5,000,000 and containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

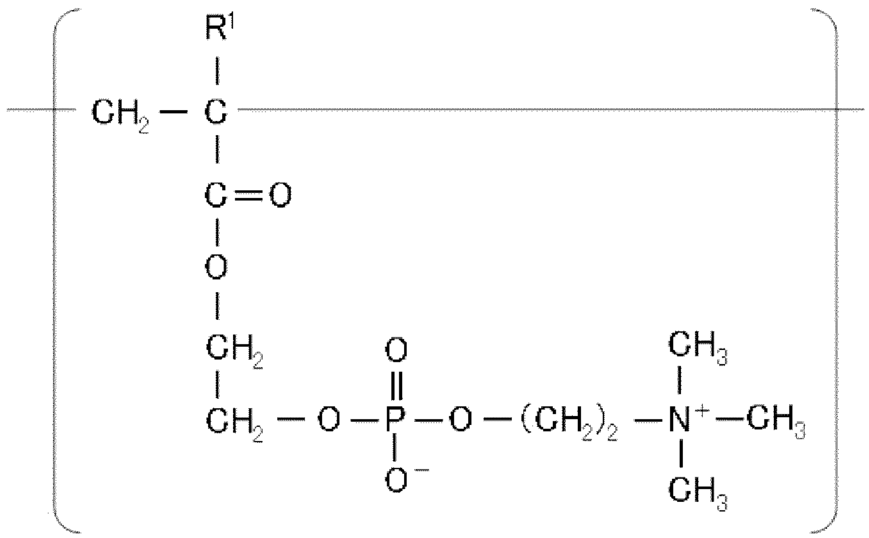

···(A)

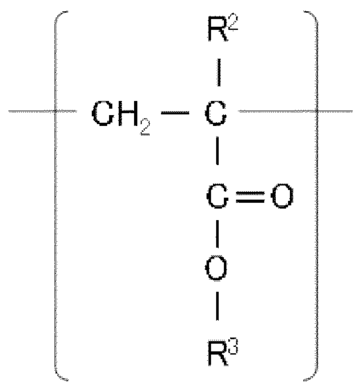

···(B1)

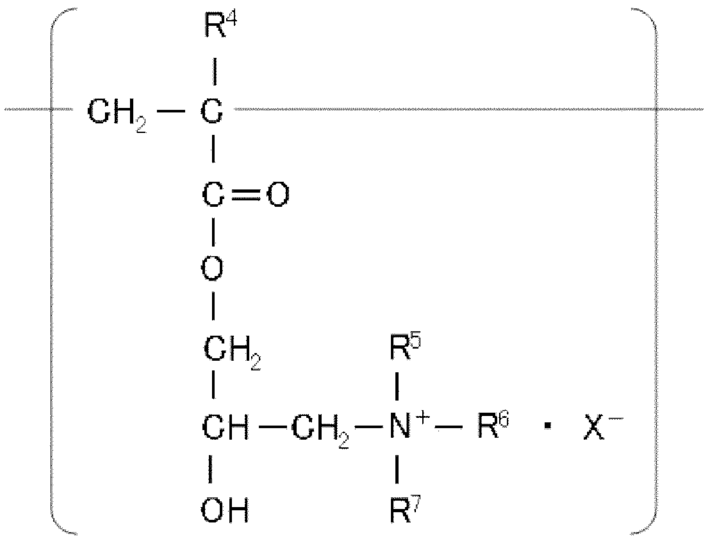

···(B2)

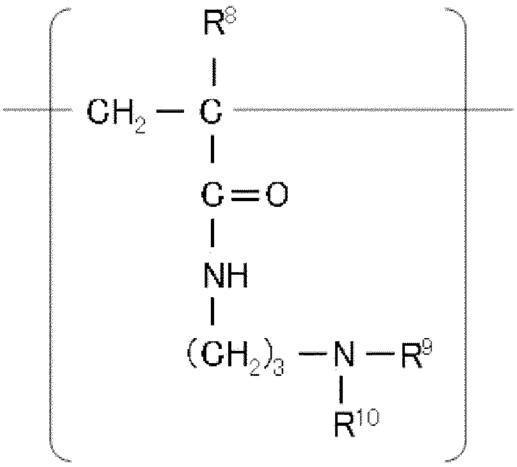

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

The present invention is also directed to a use of a copolymer for producing a biofilm formation-preventing agent, the copolymer having a weight-average molecular weight of from 10,000 to 5,000,000 and containing:

10 mol% to 90 mol% of a constituent unit (A) based on 2-(meth)acryloyloxyethyl phosphorylcholine; and
90 mol% to 10 mol% of at least one kind of constituent unit selected from the group consisting of a constituent unit (B1) based on an alkyl group-containing (meth)acrylic monomer, a constituent unit (B2) based on a quaternary ammonium group-containing (meth)acrylic monomer, and a constituent unit (B3) based on a (meth)acrylamide-based monomer:

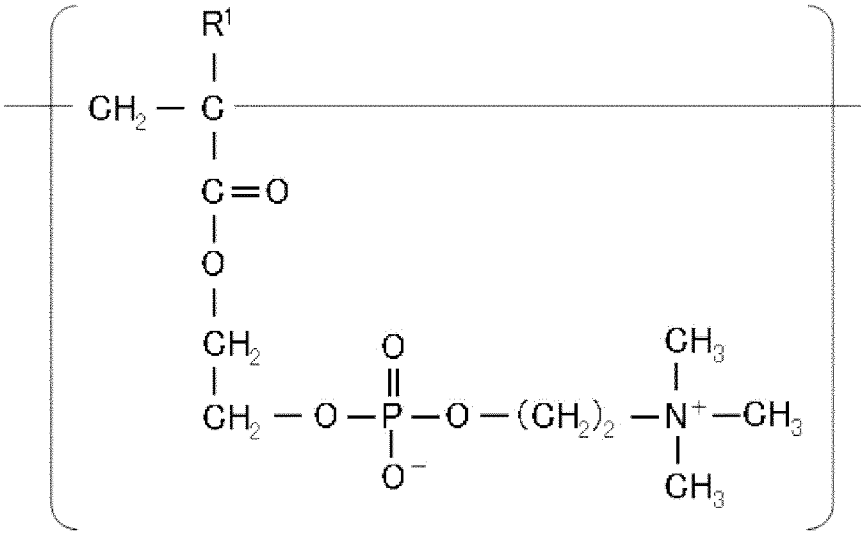

・・・(A)

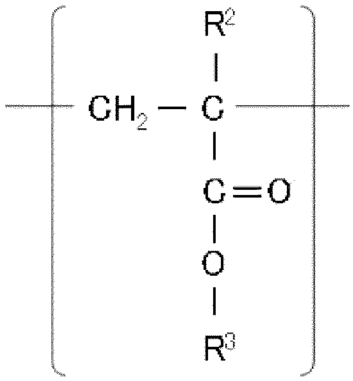

・・・(B1)

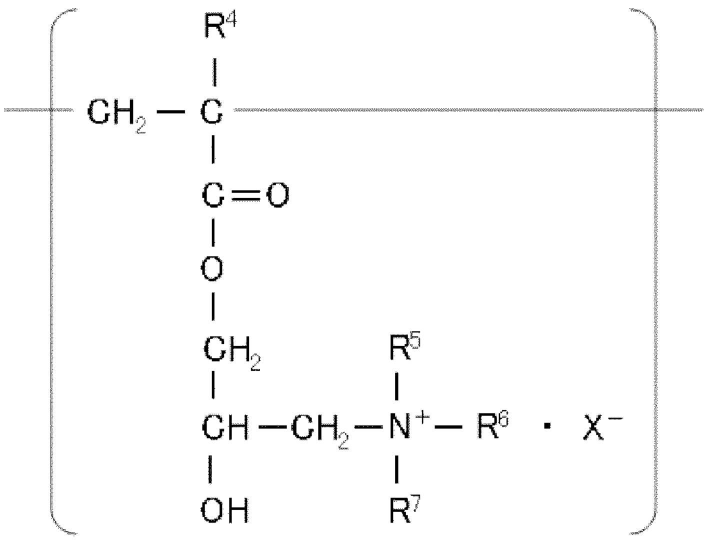

・・・(B2)

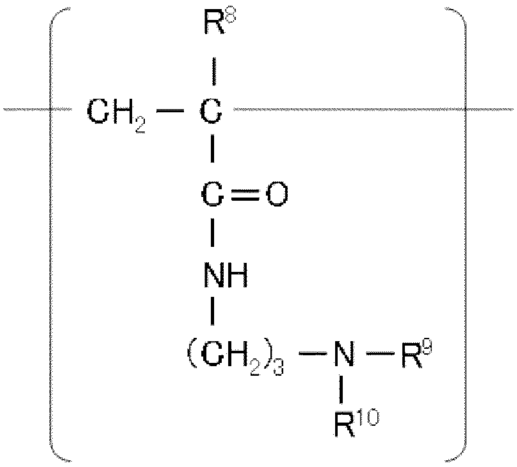

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

Examples

The effects of the biofilm formation-preventing agent, the composition for oral use containing the biofilm formation-preventing agent, and the biofilm formation-preventing method of the present invention are specifically described below by way of Examples and Comparative Examples . Copolymers and a polymer used in Examples and Comparative Examples are as described below.

MPC polymer (1): A 2-methacryloyloxyethyl phosphorylcholine-butyl methacrylate copolymer [copolymerization composition ratio (molar ratio): 80/20, weight-average molecular weight: 600,000] obtained by performing polymerization in conformity with a method described in Examples of JP 11-035605 A.

MPC polymer (2): A 2-methacryloyloxyethyl phosphorylcholine-butyl methacrylate copolymer [copolymerization composition ratio (molar ratio): 30/70, weight-average molecular weight: 142,000] obtained by performing polymerization in conformity with a method described in Examples of JP 2004-196868 A.

MPC polymer (3): A 2-methacryloyloxyethyl phosphorylcholine-stearyl methacrylate copolymer [copolymerization composition ratio (molar ratio): 33/67, weight-average molecular weight: 164,000] obtained by performing polymerization in conformity with a method described in Examples of JP 2004-196868 A.

MPC polymer (4): A 2-methacryloyloxyethyl phosphorylcholine-2-hydroxy-3-methacryloyloxypropyltrimethylammonium chloride copolymer [copolymerization composition ratio (molar ratio): 70/30, weight-average molecular weight: 450,000] obtained by performing polymerization in conformity with a method described in Examples of JP 2004-189678 A.

MPC polymer (5): A 2-methacryloyloxyethyl phosphorylcholine-N,N-dimethylaminopropyl acrylamide-stearyl methacrylate copolymer [copolymerization composition ratio (molar ratio): 90/2/8, weight-average molecular weight: 820,000] obtained by performing polymerization in conformity with a method described in Examples of JP 2013-018749 A.

Homopolymer (A): A polymer of 2-methacryloyloxyethyl phosphorylcholine [weight-average molecular weight: 200,000] obtained by performing polymerization in conformity with a method described in Examples of JP 08-333421 A.

[Examples 1-1 to 1-35, Comparative Examples 1-1 to 1-15, and References 1 to 5]

Compositions for oral use having component formulations shown in Tables 1 to 3 were prepared in accordance with an ordinary method.

<Evaluations of Adsorptivity and Retentivity of Drug>

1 mL of each of the compositions for oral use of References 1 to 5, Examples 1-1 to 1-35, and Comparative Examples 1-1 to 1-15 was brought into contact with 0.04 g of hydroxyapatite powder serving as a tooth surface model (Bio-Gel HTP Hydroxyapatite, manufactured by Bio-Rad Laboratories, Inc.) for 10 minutes. The resultant was centrifuged (at 3,500 rpm for 1 minute), and a supernatant was recovered. The supernatant was filtered with a filter ("ADVANTEC"

Cellulose Acetate manufactured by Toyo Roshi Kaisha, Ltd., pore diameter: 0.45 μm), and the amount of a drug in the supernatant was calculated by determining the amount of the drug through liquid chromatography. Then, the non-adsorption ratio of the drug was calculated from the following equation 1.

Non-adsorption ratio (%) of drug=amount of drug in supernatant/blending amount of drug×100··Equation 1

Next, 1 mL of water was added to the hydroxyapatite powder remaining after the recovery of the supernatant, and the mixture was vigorously stirred. After that, the mixture was centrifuged (at 3,500 rpm for 1 minute), and a supernatant after stirring was recovered.

Further, the supernatant was repeatedly washed three times. After that, 5 mL of purified water was added to the remaining hydroxyapatite powder, and the mixture was treated with an ultrasonic wave for 30 minutes. Thus, the drug adsorbed to the powder was extracted (recovery of an extract).

Finally, the supernatant after stirring and the extract thus recovered were each filtered with a filter ("ADVANTEC" Cellulose Acetate manufactured by Toyo Roshi Kaisha, Ltd., pore diameter: 0.45 μm), and the amount of the drug in the supernatant after stirring and the amount of the drug in the extract were each calculated by determining the amount of the drug through liquid chromatography. Then, the adsorption retention ratio of the drug was calculated from the following equation 2.

Adsorption retention ratio (%) of drug=amount of drug in extract/amount of drug in supernatant after stirring×100··Equation 2

Whether or not a composition for oral use had a controlling effect on the amount of a drug to be adsorbed to a tooth surface (excess adsorption-suppressing effect) and a drug sustained release effect was judged on the basis of the following criterion: when the total value of the non-adsorption ratio (%) and adsorption retention ratio (%) of the drug calculated as described above was more than a total value in the corresponding one of the references (References 1 to 5), the composition was judged to have such effects.

Table 1

| | | Reference 1 | Reference 2 | Reference 3 | Reference 4 | Reference 5 |
|---|---|---|---|---|---|---|
| Component formulation of composition for oral use (mass%) | Benzethonium chloride | 0.01 | | | | |
| | Chlorhexidine hydrochloride | | 0.05 | | | |
| | Isopropyl methylphenol | | | 0.10 | | |
| | dl-α-Tocopherol acetate | | | | 1.00 | |
| | Dipotassium glycyrrhizinate | | | | | 0.20 |
| | Purified water | 99.99 | 99.95 | 99.90 | 99.00 | 99.80 |
| | Total | 100.00 | 100.00 | 100.00 | 100.00 | 100.00 |

(continued)

| | | Reference 1 | Reference 2 | Reference 3 | Reference 4 | Reference 5 |
|---|---|---|---|---|---|---|
| Evaluation | Non-adsorption ratio (%) of drug | 76 | 75 | 74 | 70 | 72 |
| | Adsorption retention ratio (%) of drug | 27 | 24 | 25 | 25 | 26 |
| | Total value of non-adsorption ratio (%) and adsorption retention ratio (%) | 103 | 99 | 99 | 95 | 98 |

Table 2A

| | | Example 1-1 | Example 1-2 | Example 1-3 | Example 1-4 | Example 1-5 | Example 1-6 | Example 1-7 | Example 1-8 | Example 1-9 | Example 1-10 | Example 1-11 | Example 1-12 | Example 1-13 | Example 1-14 | Example 1-15 |
|---|---|---|---|---|---|---|---|---|---|---|---|---|---|---|---|---|
| Component formulation of composition for oral use (mass%) | Polymer (1) | 5.00 | 2.50 | 0.50 | 0.25 | | | | | | | | | | | |
| | Polymer (2) | | | | | 5.00 | 1.00 | 0.50 | 0.05 | | | | | | | |
| | Polymer (3) | | | | | | | | | 0.50 | 0.05 | | | | | |
| | Polymer (4) | | | | | | | | | | | 0.50 | 0.05 | | | |
| | Polymer (5) | | | | | | | | | | | | | 1.00 | 0.50 | 0.05 |
| | Benzethonium chloride | 0.01 | 0.01 | 0.01 | 0.01 | 0.01 | 0.01 | 0.01 | 0.01 | 0.01 | 0.01 | 0.01 | 0.01 | 0.01 | 0.01 | 0.01 |
| | Chlorhexidine hydrochloride | | | | | | | | | | | | | | | |
| | Isopropyl methylphenol | | | | | | | | | | | | | | | |
| | dl-α-Tocopherol acetate | | | | | | | | | | | | | | | |
| | Dipotassium glycyrrhizinate | | | | | | | | | | | | | | | |
| | Ethanol | | | | | | | | | | | | | | | |
| | Purified water | 94.99 | 97.49 | 99.49 | 99.74 | 94.99 | 98.99 | 99.49 | 99.94 | 99.49 | 99.94 | 99.49 | 99.94 | 98.99 | 99.49 | 99.94 |
| | Total | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |

(continued)

| | | Example 1-1 | Example 1-2 | Example 1-3 | Example 1-4 | Example 1-5 | Example 1-6 | Example 1-7 | Example 1-8 | Example 1-9 | Example 1-10 | Example 1-11 | Example 1-12 | Example 1-13 | Example 1-14 | Example 1-15 |
|---|---|---|---|---|---|---|---|---|---|---|---|---|---|---|---|---|
| Evaluation | Non-adsorption ratio (%) of drug | 84 | 82 | 77 | 76 | 90 | 85 | 83 | 80 | 80 | 75 | 80 | 79 | 82 | 80 | 77 |
| | Adsorption retention ratio (%) of drug | 36 | 35 | 32 | 31 | 40 | 37 | 35 | 33 | 33 | 33 | 34 | 33 | 36 | 35 | 33 |
| | Total value of non-adsorption ratio (%) and adsorption retention ratio (%) | 120 | 117 | 109 | 107 | 130 | 122 | 118 | 113 | 113 | 108 | 114 | 112 | 118 | 115 | 110 |

Table 2B

| | | Example 1-16 | Example 1-17 | Example 1-18 | Example 1-19 | Example 1-20 | Example 1-21 | Example 1-22 | Example 1-23 | Example 1-24 | Example 1-25 | Example 1-26 | Example 1-27 | Example 1-28 | Example 1-29 | Example 1-30 | Example 1-31 | Example 1-32 | Example 1-33 | Example 1-34 | Example 1-35 |
|---|---|---|---|---|---|---|---|---|---|---|---|---|---|---|---|---|---|---|---|---|---|
| Component formulation of composition for oral use (mass%) | Polymer (1) | 1.00 | 1.00 | 1.00 | 1.00 | | | | | | | | | | | | | | | | |
| | Polymer (2) | | | | | 0.50 | 0.50 | 0.50 | 0.50 | | | | | | | | | | | | |
| | Polymer (3) | | | | | | | | | 0.10 | 0.10 | 0.10 | 0.10 | | | | | | | | |
| | Polymer (4) | | | | | | | | | | | | | 0.50 | 0.80 | 1.00 | 1.00 | | | | |
| | Polymer (5) | | | | | | | | | | | | | | | | | 0.50 | 1.00 | 1.00 | 1.50 |
| | Benzethonium chloride | | | | | | | | | | | | | | | | | | | | |
| | Chlorhexidine hydrochloride | 0.05 | | | | 0.05 | | | | 0.05 | | | | 0.05 | | | | 0.05 | | | |
| | Isopropyl methylphenol | | 0.10 | | | | 0.10 | | | | 0.10 | | | | 0.10 | | | | 0.10 | | |
| | dl-α-Tocopherol acetate | | | 1.00 | | | | 1.00 | | | | 1.00 | | | | 1.00 | | | | 1.00 | |
| | Dipotassium glycyrrhizinate | | | | 0.20 | | | | 0.20 | | | | 0.20 | | | | 0.20 | | | | 0.20 |
| | Ethanol | 5.00 | 10.00 | | | 10.00 | | | | 10.00 | 5.00 | | | | | | | | | | |
| | Purified water | 93.95 | 88.90 | 98.00 | 98.80 | 89.45 | 99.40 | 98.50 | 99.30 | 89.85 | 94.80 | 98.90 | 99.70 | 99.45 | 99.10 | 98.00 | 98.80 | 99.45 | 98.90 | 98.00 | 98.30 |
| | Total | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Evaluation | Non-adsorption ratio (%) of drug | 80 | 81 | 75 | 78 | 83 | 82 | 80 | 80 | 79 | 80 | 78 | 78 | 80 | 80 | 79 | 78 | 78 | 79 | 76 | 80 |
| | Adsorption retention ratio (%) of drug | 32 | 33 | 30 | 31 | 33 | 34 | 31 | 32 | 33 | 32 | 31 | 30 | 32 | 33 | 33 | 33 | 33 | 31 | 31 | 32 |
| | Total value of non-adsorption ratio (%) and adsorption retention ratio (%) | 112 | 114 | 105 | 109 | 116 | 116 | 111 | 112 | 112 | 112 | 109 | 108 | 112 | 113 | 112 | 111 | 111 | 110 | 107 | 112 |

Table 3

| | | Com-para tive Example 1-1 | Compara tive Example 1-2 | Compara tive Example 1-3 | Compara tive Example 1-4 | Compara tive Example 1-5 | Compara tive Example 1-6 | Compara tive Example 1-7 | Compara tive Example 1-8 | Compara tive Example 1-9 | Compara tive Example 1-10 | Compara tive Example 1-11 | Compara tive Example 1-12 | Compara tive Example 1-13 | Compara tive Example 1-14 | Compara tive Example 1-15 |
|---|---|---|---|---|---|---|---|---|---|---|---|---|---|---|---|---|
| Compo-nent for-mulation of compositi on for oral use (mass%) | Homopolymer (A) | 0.50 | | | 1.00 | | | 1.00 | | | 1.00 | | | 1.00 | | |
| | Hydroxyethyl cellulose | | 0.50 | | | 0.50 | | | 0.50 | | | 0.50 | | | 0.50 | |
| | Polyethylene glycol | | | 0.50 | | | 0.50 | | | 0.50 | | | 0.50 | | | 0.50 |
| | Benzethonium chloride | 0.01 | 0.01 | 0.01 | | | | | | | | | | | | |
| | Chlorhexidin e hydrochlorid e | | | | 0.05 | 0.05 | 0.05 | | | | | | | | | |
| | Isopropyl methylphenol | | | | | | | 0.10 | 0.10 | 0.10 | | | | | | |
| | dl-α-Toco-phe rol ace-tate | | | | | | | | | | 1.00 | 1.00 | 1.00 | | | |
| | Dipotassium glycyrrhizin ate | | | | | | | | | | | | | 0.20 | 0.20 | 0.20 |
| | Purified water | 99.49 | 99.49 | 99.49 | 98.95 | 99.45 | 99.45 | 98.90 | 99.40 | 99.40 | 98.00 | 98.50 | 98.50 | 98.80 | 99.30 | 99.30 |
| | Total | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |

(continued)

| | | Com-para tive Example 1-1 | Compara tive Example 1-2 | Compara tive Example 1-3 | Compara tive Example 1-4 | Compara tive Example 1-5 | Compara tive Example 1-6 | Compara tive Example 1-7 | Compara tive Example 1-8 | Compara tive Example 1-9 | Compara tive Example 1-10 | Compara tive Example 1-11 | Compara tive Example 1-12 | Compara tive Example 1-13 | Compara tive Example 1-14 | Compara tive Example 1-15 |
|---|---|---|---|---|---|---|---|---|---|---|---|---|---|---|---|---|
| Evaluation | Non-adsorption ratio (%) of drug | 73 | 68 | 71 | 70 | 73 | 74 | 75 | 70 | 72 | 70 | 68 | 68 | 71 | 66 | 69 |
| | Adsorption retention ratio (%) of drug | 28 | 25 | 26 | 26 | 23 | 25 | 24 | 25 | 25 | 25 | 24 | 25 | 26 | 26 | 25 |
| | Total value of non-adsorption ratio (%) and adsorption retention ratio (%) | 101 | 93 | 97 | 96 | 96 | 99 | 99 | 95 | 97 | 95 | 92 | 93 | 97 | 92 | 94 |

As a result, all of Examples 1-1 to 1-35 satisfied the above-mentioned criterion.

That is, the compositions for oral use of Examples 1-1 to 1-35 each had a controlling effect on the amount of a drug to be adsorbed to a tooth surface (excess adsorption-suppressing effect) and a drug sustained release effect.

The above-mentioned results are described in more detail below.

When References 1 to 5 were each compared to Examples, the excess adsorption amount of the drug was large, and hence the non-adsorption ratio of the drug was low, and the adsorption retention ratio of the drug was also low.

In contrast, as shown in each of Examples 1-1 to 1-15, the composition for oral use containing the biofilm formation-preventing agent of the present invention was able to control the total value of the non-adsorption ratio and the adsorption retention ratio, i.e., the adsorption amount of its drug through an increase or reduction in content of its polymer, and had sustained releasability by virtue of the high total value of the non-adsorption ratio and the adsorption retention ratio. Accordingly, it was confirmed that the composition improved a biofilm formation-preventing effect, and hence was able to achieve the suppression of coaggregation in which any other microbe adhered to a microbe that had already adhered to an oral cavity, and the prevention of stickiness in the oral cavity.

Meanwhile, when the non-adsorption ratio of the drug and the adsorption retention ratio of the drug in each of Comparative Examples 1-1 to 1-15 were compared to the non-adsorption ratio of the drug and the adsorption retention ratio of the drug in the corresponding one of References 1 to 5, no differences were observed, and hence it was confirmed that each of Comparative Examples was free of an adsorption amount-controlling effect and sustained releasability.

As can be seen from the foregoing, in each of the biofilm formation-preventing agent, the composition for oral use containing the biofilm formation-preventing agent, and the biofilm formation-preventing method of the present invention, an excellent adsorption amount-controlling effect and an excellent sustained release effect are exhibited, and a biofilm formation-preventing effect is improved, and hence the suppression of coaggregation in which any other microbe adheres to a microbe that has already adhered to an oral cavity, and the prevention of stickiness in the oral cavity can be achieved.

## Industrial Applicability

There can be provided a biofilm formation-preventing agent, a composition for oral use, and a biofilm formation-preventing method in each of which the sustainability of an effect of a drug is improved, and an oral environment is kept satisfactory in a sustainable manner without an increase in drug concentration or treatment time, and hence the maturation of dental plaque into a biofilm is prevented, and a composition for oral use that can prevent the deterioration of the oral environment through a biofilm formation-preventing effect to prevent stickiness in an oral cavity.

## Claims

1. A biofilm formation-preventing agent, comprising a copolymer having a weight-average molecular weight of from 10,000 to 5,000,000 and containing:

   10 mol% to 90 mol% of a constituent unit (A) based on 2-(meth)acryloyloxyethyl phosphorylcholine; and
   90 mol% to 10 mol% of at least one kind of constituent unit selected from the group consisting of a constituent unit (B1) based on an alkyl group-containing (meth) acrylic monomer, a constituent unit (B2) based on a quaternary ammonium group-containing (meth)acrylic monomer, and a constituent unit (B3) based on a (meth)acrylamide-based monomer:

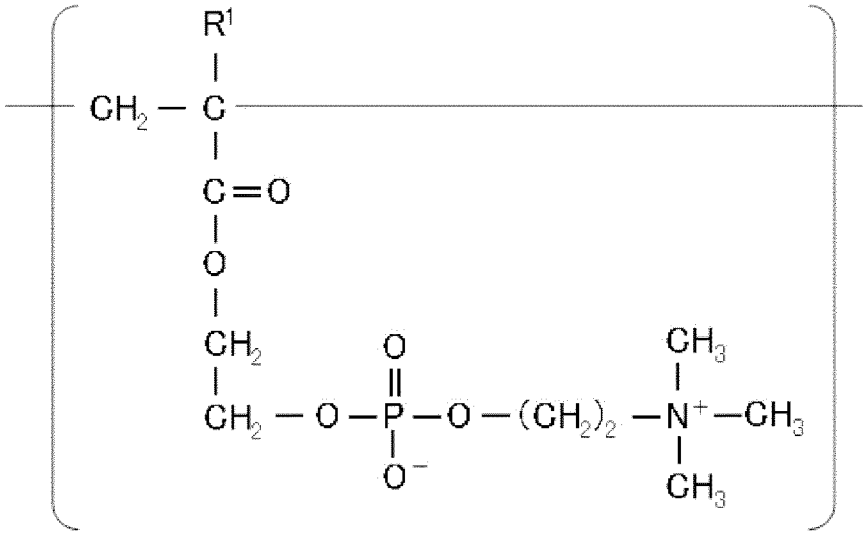

・・・(A)

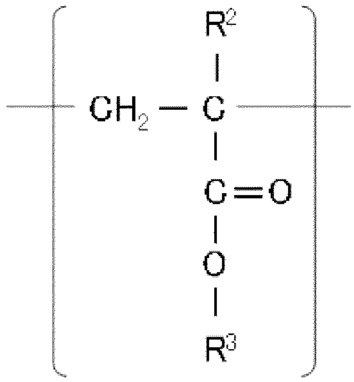

・・・(B1)

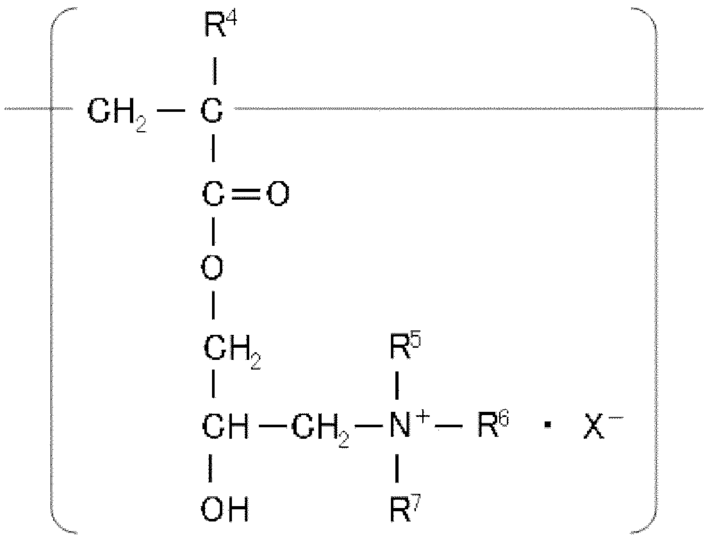

・・・(B2)

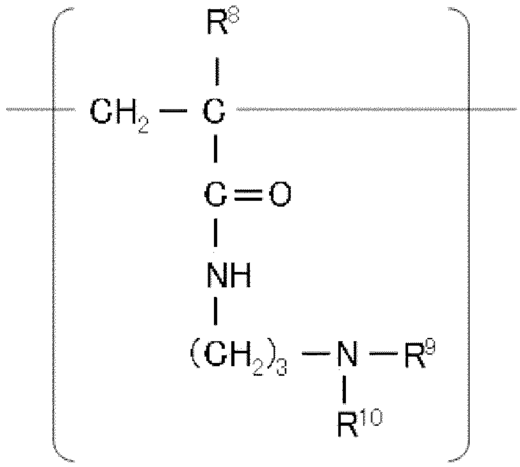

···(B3)

in the formula (A), $R^1$ represents a hydrogen atom or a methyl group, in the formula (B1), $R^2$ represents a hydrogen atom or a methyl group, and $R^3$ represents an alkyl group having 4 to 18 carbon atoms, in the formula (B2), $R^4$ represents a hydrogen atom or a methyl group, $R^5$, $R^6$, and $R^7$ each independently represent a hydrogen atom or an alkyl group having 1 to 8 carbon atoms, and $X^-$ represents a halogen ion or an acid residue, and in the formula (B3), $R^8$ represents a hydrogen atom or a methyl group, and $R^9$ and $R^{10}$ each independently represent a hydrogen atom or an alkyl group having 1 to 6 carbon atoms.

**2.** A biofilm formation-preventing agent according to claim 1, wherein the constituent unit (A) comprises a constituent unit based on 2-(meth)acryloyloxyethyl phosphorylcholine, and the constituent unit (B1) comprises a constituent unit based on butyl methacrylate.

**3.** A biofilm formation-preventing agent according to claim 1, wherein the constituent unit (A) comprises a constituent unit based on 2-(meth)acryloyloxyethyl phosphorylcholine, and the constituent unit (B1) comprises a constituent unit based on stearyl methacrylate.

**4.** A biofilm formation-preventing agent according to claim 1, wherein the constituent unit (A) comprises a constituent unit based on 2-(meth)acryloyloxyethyl phosphorylcholine, and the constituent unit (B2) comprises a constituent unit based on 2-hydroxy-3-methacryloyloxypropyltrimethylammonium chloride.

**5.** A biofilm formation-preventing agent according to claim 1, wherein the constituent unit (A) comprises a constituent unit based on 2-(meth)acryloyloxyethyl phosphorylcholine, the constituent unit (B1) comprises a constituent unit based on stearyl methacrylate, and the constituent unit (B3) comprises a constituent unit based on N,N-dimethylaminopropyl acrylamide.

**6.** A composition for oral use, comprising:

the biofilm formation-preventing agent of any one of claims 1 to 5; and
one or two or more kinds of drugs selected from the group consisting of a disinfectant (C), an anti-inflammatory agent (D), and a vitamin (E).

## INTERNATIONAL SEARCH REPORT

International application No.
PCT/JP2016/072955

A. CLASSIFICATION OF SUBJECT MATTER
*A61K8/81*(2006.01)i, *A61Q11/00*(2006.01)i

According to International Patent Classification (IPC) or to both national classification and IPC

B. FIELDS SEARCHED

Minimum documentation searched (classification system followed by classification symbols)
A61K8/81, A61Q11/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/MEDLINE/KOSMET(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2011-153101 A (NOF Corp.), 11 August 2011 (11.08.2011), claims 2, 6; paragraphs [0027] to [0038] (Family: none) | 1-6<br>1-6 |
| X<br>Y | JP 2006-273767 A (Nissan Soap Co., Ltd.), 12 October 2006 (12.10.2006), claims; paragraphs [0013], [0022] to [0023], [0025]; examples (Family: none) | 1-6<br>1-6 |
| Y | JP 2003-180801 A (Yoichiro MIYAKE), 02 July 2003 (02.07.2003), claims; paragraphs [0013] to [0018], [0022] (Family: none) | 1-6 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 October 2016 (13.10.16) | 01 November 2016 (01.11.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/072955

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2016-37455 A (Earth Chemical Co., Ltd.), 22 March 2016 (22.03.2016), claim 2 (Family: none) | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015155410 A
- JP 2008120753 A
- JP 2009102283 A
- JP 2006273767 A
- JP 2011153101 A
- JP 11035605 A
- JP 2004196868 A
- JP 2004189678 A
- JP 2013018749 A
- JP 8333421 A